(19) Europäisches Patentamt
European Patent Office
Office européen des brevets

(11) EP 2 515 886 B1

(12) EUROPÄISCHE PATENTSCHRIFT

(45) Veröffentlichungstag und Bekanntmachung des Hinweises auf die Patenterteilung:
**11.04.2018 Patentblatt 2018/15**

(21) Anmeldenummer: **10790565.5**

(22) Anmeldetag: **14.12.2010**

(51) Int Cl.:
***A61K 9/70*** *(2006.01)* ***A61K 31/27*** *(2006.01)*
***A61K 31/445*** *(2006.01)* ***A61K 31/55*** *(2006.01)*

(86) Internationale Anmeldenummer:
**PCT/EP2010/069654**

(87) Internationale Veröffentlichungsnummer:
**WO 2011/076621 (30.06.2011 Gazette 2011/26)**

(54) **TRANSDERMALES THERAPEUTISCHES SYSTEM ZUR VERABREICHUNG VON RIVASTIGMIN ODER DESSEN DERIVATEN**

TRANSDERMAL THERAPEUTIC SYSTEM FOR ADMINISTERING RIVASTIGMINE OR DERIVATIVES THEREOF

SYSTÈME THÉRAPEUTIQUE TRANSDERMIQUE SERVANT À ADMINISTRER DE LA RIVASTIGMINE OU SES DÉRIVÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2009 EP 09180413**
**25.02.2010 EP 10154648**

(43) Veröffentlichungstag der Anmeldung:
**31.10.2012 Patentblatt 2012/44**

(73) Patentinhaber: **Luye Pharma AG**
**83714 Miesbach (DE)**

(72) Erfinder:
- **PRINZ, Heike**
  **82008 Unterhaching (DE)**
- **SCHURAD, Björn**
  **81667 München (DE)**
- **BECKERT, Thomas**
  **88447 Birkenhard (DE)**
- **LINDER, Kristina**
  **83088 Kiefersfelden (DE)**

(74) Vertreter: **Kalhammer, Georg et al**
**Lederer & Keller**
**Patentanwälte Partnerschaft mbB**
**Unsöldstrasse 2**
**80538 München (DE)**

(56) Entgegenhaltungen:
**WO-A1-2007/064407** **US-A1- 2004 086 552**
**US-A1- 2007 259 028** **US-A1- 2008 044 461**
**US-B1- 6 689 379**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 515 886 B1

## Beschreibung

Gegenstand der vorliegenden Anmeldung ist ein System zur transdermalen Verabreichung von Rivastigmin, dessen physiologisch verträglichem Salz, Hydrat oder Solvat für therapeutische Zwecke.

Bei Rivastigmin handelt es sich um das Phenylcarbamat (S)-N-ethyl-3-[(1-dimethylamino)ethyl]-N-methyl-phenyl-carbamat der Formel I.

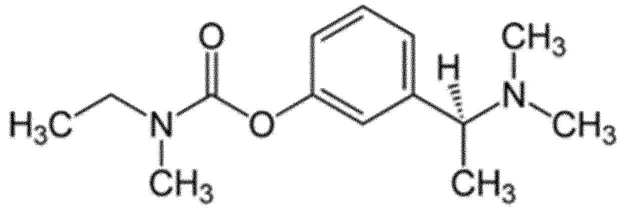

(I)

Es ist ein im zentralen Nervensystem wirkender Cholinesteraseinhibitor und ist daher ein Wirkstoff zur Behandlung von Alzheimer und Parkinsondemenz.

Rivastigmin kann als freie Base, aber auch als Säureadditionssalz, Hydrat, Solvat oder als sonstiges Derivat vorliegen. Diese Derivate sind, sofern nicht anders beschrieben, in der vorliegenden Erfindung mit der Bezeichnung "Rivastigmin" umfasst.

Eine bevorzugte Darreichungsform von Rivastigmin ist die perkutane Verabreichung mittels eines transdermalen therapeutischen Systems, also ein Transdermalpflaster. Ein Transdermalpflaster ist typischerweise eine kleine selbstklebende Bandage, welche den abzugebenden Wirkstoff enthält. Diese Bandagen können verschiedene Formen und Größen haben. Der einfachste Typ ist ein Klebe-Monolit, welcher einen Wirkstoffvorrat (Reservoir) auf einem Träger (Deckschicht) umfasst. Das Reservoir wird typischerweise aus dem Wirkstoff in einem pharmazeutisch akzeptablen, druckempfindlichen Klebstoff gebildet und steht mit der Hautoberfläche in Kontakt, wodurch der Wirkstoff durch transdermale Diffusion in den Körper des Patienten abgegeben wird.

Komplexere Pflaster sind Mehrfachlaminate oder Pflaster mit Wirkstoffvorrat, in welchen zwischen dem Reservoir und der Haut eine weitere Klebeschicht angeordnet sein kann.

Eine Darreichungsform über Transdermalpflaster von Rivastigmin wurde bereits im Grundpatent zu Rivastigmin, der GB 2203040 beschrieben. Das darin offenbarte Transdermalpflaster besteht aus einer Deckschicht und einer das Reservoir bildenden Schicht. Neben dem Wirkstoff Rivastigmin sind in dem Reservoir ein hydrophiles Polymer, ein nichtquellendes Acrylatpolymer und ein Weichmacher enthalten.

Nach Veröffentlichung der GB 2203040 wurden weitere transdermale therapeutische Systeme (TTS), die unter anderem Rivastigmin als Wirkstoff enthalten, entwickelt und beschrieben. In der WO 02/03969 ist ein TTS beschrieben, bei dem die wirkstoffenthaltende Matrixschicht (Reservoir) zusätzlich hochdisperses Siliziumdioxid zur Erhöhung der Hautpermeation enthält.

In der DE 199 18 106 enthält das Reservoir ein haftklebendes Polymer, welches Acrylsäure oder Methacrylsäureeinheiten mit einem definierten Gehalt an Carboxylgruppen aufweist, wodurch die Wasseraufnahmefähigkeit sowie die Toleranz saurer Polyacrylhaftkleber gegenüber Feuchtigkeit erhöht werden soll.

Die WO 2007/064407 A1 offenbart ein TTS mit einer auf Silikon basierenden Klebeschicht, wodurch eine Verbesserung hinsichtlich der Klebeeigenschaften, der Verträglichkeit und der Sicherheit bei der Rivastigmintherapie erreicht werden soll. Gemäß WO 2007/064407 A1 ist es besonders bevorzugt, dass die Reservoirschicht ein Antioxidationsmittel enthält (Seite 7, 4. Absatz). Entsprechend enthalten alle Formulierungen in den Beispielen das Antioxidationsmittel Vitamin E. Das dort verwendete Durotak® 387-2353 ist ein Polyacrylat, das Carboxylgruppen aufweist. Die Reservoirschicht soll nach WO 2007/064407 A1 auch als Penetrationsverbesserer verschiedene Substanzen wie z. B. Glycerin, Fettsäuren, etc. enthalten (Seite 7, 5. Absatz). Diese Substanzen enthalten meist freie Hydroxyl- oder Carboxylgruppen, die damit in der Polymermatrix der Reservoirschicht vorhanden sind. WO 2007/064407 A1 beschäftigt sich nicht besonders mit der Stabilität von Rivastigmin. Insbesondere lehrt WO 2007/064407 A1 nicht, bestimmte Polymere für die Polymermatrix der Reservoirschicht auszuwählen, um einem Abbau von Rivastigmin vorzubeugen.

US 2008/0044461 A1 offenbart TTS-Formulierungen mit Donepezil (siehe Beispiele). Rivastigmin wird auch erwähnt (Anspruch 7). US 2008/0044461 A1 offenbart keine Wirkstoffschicht mit einer Polymermatrix. Vielmehr wird die Freisetzung über eine Membran gesteuert (sog. Membranpflaster), nicht durch ein Polymergerüst, in das der Wirkstoff eingebettet ist (sog. Matrixpflaster). Zudem ist es ein essentielles Merkmal von US 2008/0044461 A1, dass die Reservoirschicht einen Gelbildner und einen Permeationsverstärker enthält (siehe Anspruch 1). Als Permeationsverstärker werden Alkohole eingesetzt (siehe [0053]). Als Gelbildner werden Cellulosepolymere eingesetzt (siehe [0055]). Sowohl bei den Permeationsverstärkern als auch bei den Gelbildnern handelt es sich somit um Verbindungen mit freien Hydroxylgruppen, die im TTS in der Reservoirschicht vorhanden sind.

US 2007/0259028 A1 offenbart TTS-Formulierungen mit Donepezil (siehe Beispiele). Rivastigmin wird auch erwähnt (Anspruch 3). Es ist ein essentielles Merkmal von US 2007/0259028 A1, dass die Reservoirschicht einen mehrwertigen Alkohol enthält, z. B. Glycerin. Gemäß US 2007/0259028 A1 sind also zwangsläufig freie Hydroxylgruppen in der Polymermatrix der Reservoirschicht vorhanden.

US 2004/0086552 A1 offenbart TTS-Formulierungen mit einem Wirkstoff, der aus einer sehr langen Liste ausgewählt sein kann (siehe [0070] bis [0095]). Es werden sowohl Matrixpflaster als auch "Membranpflaster" offenbart (siehe [0057] bzw. [0058]). Für die Matrixpflaster lehrt US 2004/0086552 A1 nicht, bestimmte Polymere für die Matrix auszuwählen, um den Wirkstoff zu stabilisieren.

US 6,689,379 B1 offenbart TTS-Formulierungen mit einer besonderen Klebeschicht. Rivastigmin wird auch als möglicher Wirkstoff erwähnt. Die Wirkstoffschicht soll vorzugsweise eine Verbindung mit Hydroxylgruppen enthalten, siehe Anspruch 10. US 6,689,379 B1 lehrt nicht, bestimmte Polymere für die Polymermatrix der Reservoirschicht auszuwählen, um einem Abbau von Rivastigmin vorzubeugen.

Das Patent EP 1 047 409 berichtet jedoch von einem generellen Problem bei der Verabreichung von Rivastigmin durch ein TTS. Es wurde gefunden, dass der Wirkstoff gerade in Gegenwart von Sauerstoff zersetzungsanfällig ist. In der transdermalen Zusammensetzung welche in der GB 2203040 offenbart ist, zersetzt sich gemäß der Offenbarung in EP 1 047 409 das Rivastigmin auch trotz der Bildung einer geschlossenen Polymermatrix um den Wirkstoff und einer luftdichten Verpackung der Zusammensetzung. In der EP 1 047 409 wird das Problem der geringen Stabilität von Rivastigmin dadurch gelöst, dass der pharmazeutischen Zusammensetzung ein Antioxidationsmittel zugesetzt wird.

Die Aufgabe der vorliegenden Erfindung ist es daher therapeutische Zusammensetzungen enthaltend Rivastigmin für die transdermale Verabreichung zu finden, welche auch ohne Zusatz von Antioxidationsmitteln hinreichend stabil sind.

Es wurde überraschenderweise gefunden, dass Rivastigmin in Transdermalpflastern hinreichend stabil ist, wenn die Polymermatrix des Reservoirs weder Hydroxylgruppen noch Carboxylgruppen aufweist. Die vorliegende Erfindung beruht unter anderem darauf, spezielle Polymere für die Polymermatrix auszuwählen, um dadurch einen Abbau von Rivastigmin zu verhindern bzw. zu minimieren.

Die vorliegende Erfindung stellt daher ein hinreichend stabiles TTS enthaltend Rivastigmin und ein Verfahren zur Herstellung desselben bereit. Weiterhin stellt die Erfindung die Verwendung von Polymeren oder Copolymeren, welche weder Hydroxylgruppen noch Carboxylgruppen aufweisen, in einem TTS enthaltend Rivastigmin und ein TTS zur Behandlung von Alzheimer und Parkinsondemenz bereit.

Ein erster Aspekt der Erfindung ist daher ein TTS zur Verabreichung von Rivastigmin, das folgende Komponenten umfasst:

a) eine Deckschicht,

b) ein auf der Deckschicht befindliches Reservoir umfassend eine Polymermatrix, in die der Wirkstoff eingebettet ist, wobei der Wirkstoff Rivastigmin oder ein physiologisch verträgliches Salz, Hydrat oder Solvat davon ist,

c) eine auf dem Reservoir befindliche Klebeschicht umfassend als Haftkleber ein erstes Polyisobutylenpolymer mit einem mittleren Molekulargewicht zwischen 30.000 g/mol und 100.000 g/mol, und ein zweites Polyisobutylenpolymer mit einem mittleren Molekulargewicht zwischen 300.000 g/mol und 500.000 g/mol; und

d) eine auf der Klebeschicht befindliche Abziehschicht,

dadurch gekennzeichnet, dass die Polymermatrix des Reservoirs weder Hydroxylgruppen noch Carboxylgruppen aufweist, und das Reservoir kein Tocopherol, kein Butylhydroxdyanisol und kein Butylhydroxytoluol enthält.

Ein zweiter Aspekt der Erfindung ist ein TTS zur Verabreichung von Rivastigmin, das folgende Komponenten umfasst:

a) eine Deckschicht,

b) ein auf der Deckschicht befindliches Reservoir umfassend eine Polymermatrix, in die der Wirkstoff eingebettet ist,

c) eine auf dem Reservoir befindliche Klebeschicht umfassend einen Haftkleber und

d) eine auf der Klebeschicht befindliche Abziehschicht,

wobei der Wirkstoff Rivastigmin oder ein physiologisch verträgliches Salz, Hydrat, oder Solvat davon ist, und wobei die Polymermatrix des Reservoirs weder Hydroxylgruppen noch Carboxylgruppen aufweist, und das Reservoir kein Tocopherol, kein Butylhydroxdyanisol und kein Butylhydroxytoluol enthält.

Eine "Polymermatrix" ist eine feste oder halbfeste Zusammensetzung mit dreidimensionaler Struktur, umfassend ein Polymer oder ein Polymergemisch. Die Polymermatrix wird auch als Polymergerüst bezeichnet, da die dreidimensionale Gerüst-Struktur in der Regel durch das Polymer oder Polymergemisch bereitgestellt wird. In die Polymermatrix können andere Stoffe, z. B. ein Wirkstoff, eingebettet sein. Vorzugsweise ist der Wirkstoff gleichmäßig in der Polymermatrix verteilt. Dem Fachmann sind sogenannte "Matrixpflaster" an sich bekannt, bei denen die Freisetzung des Wirkstoffs durch die Polymermatrix kontrolliert wird.

Der Wirkstoff Rivastigmin ist in dem erfindungsgemäßen TTS hinreichend stabil. "Hinreichend stabil" bedeutet, dass die Verunreinigungen des Wirkstoffs nach einem Monat Lagerung bei 40°C und 75 % relative Luftfeuchtigkeit insgesamt nicht mehr als 1 Gew.-% , bevorzugt nicht mehr als 0,5 Gew.-% betragen, bezogen auf den Sollgehalt an Wirkstoff in der Formulierung. Als Verunreinigungen des Wirkstoffs in der Formulierung gelten Abbauprodukte des Wirkstoffs Rivastigmin und mit dem Wirkstoff in die Formulierung eingetragene Verunreinigungen (z. B. Spuren von Zwischenprodukten aus der Herstellung des Wirkstoffs).

Die Stabilität bzw. die Menge an Verunreinigungen kann wie in dem Beispiel beschrieben bestimmt werden. Vorzugsweise ist der Gesamtgehalt der Zersetzungsprodukte / Verunreinigungen nach drei Monaten Lagerung bei 40°C und 75 % relative Luftfeuchtigkeit weniger als 1 Gew.-%, bevorzugter weniger als 0,6 Gew.-%. Es ist auch bevorzugt, dass der Gesamtgehalt der Zersetzungsprodukte / Verunreinigungen nach sechs Monaten Lagerung bei 40°C und 75 % relative Luftfeuchtigkeit weniger als 1 Gew.-% beträgt. Es ist weiterhin bevorzugt, dass der Gesamtgehalt der Verunreinigungen nach einem Monat Lagerung bei 25°C und 60 % relative Luftfeuchtigkeit weniger als 0,25 Gew.-% beträgt. Es ist weiterhin bevorzugt, dass der Gesamtgehalt der Verunreinigungen nach drei und nach sechs Monaten Lagerung bei 25°C und 60 % relative Luftfeuchtigkeit weniger als 0,5 Gew.-% beträgt. Die Angaben von "Gew.-%" Verunreinigungen beziehen sich immer auf den Sollgehalt an Wirkstoff in der Formulierung, soweit nicht anders angegeben.

Die Anwendungsdauer eines erfindungsgemäßen TTS beträgt bevorzugt etwa 24 Stunden. Eine längere Anwendungsdauer ist möglich.

Bevorzugterweise wird keinem Bestandteil des TTS ein Antioxidationsmittel zugesetzt. Es ist jedoch durchaus möglich, dass in dem TTS der vorliegenden Erfindung Antioxidationsmittel vorhanden sein können, sofern diese die Wirkungsweise des TTS nicht negativ beeinflussen. Es ist hierbei zu beachten, dass zur Stabilisierung von Rivastigmin gemäß der vorliegenden Erfindung keine Antioxidationsmittel nötig sind. Jedoch könnten Antioxidationsmittel auch für andere Zwecke in den erfindungsgemäßen TTS zum Einsatz kommen. Es ist daher möglich, wenn auch nicht bevorzugt, dass das erfindungsgemäße TTS Antioxidationsmittel enthält.

Ein "Antioxidationsmittel" im Sinne der vorliegenden Erfindung ist eine pharmazeutisch verträgliche Verbindung oder Zusammensetzung, die Oxidationsprozesse verlangsamt, hemmt, unterbricht und/oder aufhält. Antioxidationsmittel schließen insbesondere folgende Substanzen ein: Tocopherole und deren Ester, das Sesamol des Sesamöls, das Koniferylbenzoat des Benzoeharzes, die Nordihydroguajakharz- und -guajaretsäure (NDGA), die Gallate (Methyl-, Ethyl-, Propyl-, Amyl-, Butyl-, Lauryl- u.a. Gallate), das Butylhydroxdyanisol (BHT, auch Butyl-p-Kresol genannt); Ascorbinsäure und Salze und Ester davon, (z. B. Ascorbylpalmitat), Erythorbinsäure (iso-Ascorbinsäure) und Salze und Ester davon, Monothioglycerol, Natriumformaldehydsulfoxylat, Natriummetabisulfit, Natriumbisulfit, Natriumsulfit, Kaliummetabisulfit, butyliertes Hydroxyanisol, butyliertes Hydroxytoluol, Propionsäure. Typische Antioxidationsmittel sind Tocopherole wie $\alpha$-Tocopherol und dessen Ester, Butylhydroxytoluol und Butylhydroxyanisol. Der Begriff "Tocopherol" schließt auch Tocopherol-Ester ein. Ein bekanntes Tocopherol ist $\alpha$-Tocopherol. Der Begriff "$\alpha$-Tocopherol" schließt Ester von $\alpha$-Tocopherol (z. B. $\alpha$-Tocopherol-Acetat) ein.

Vorzugsweise enthält das Reservoir, bevorzugter das gesamte TTS, kein Tocopherol. In einer weiteren Ausführungsform enthält das Reservoir, vorzugsweise das gesamte TTS, kein Tocopherol und kein Butylhydroxdyanisol (BHT, auch Butyl-p-Kresol genannt). In einer weiteren Ausführungsform enthält das Reservoir, vorzugsweise das gesamte TTS, kein Tocopherol, kein Butylhydroxdyanisol und kein Butylhydroxytoluol. In einer besonderen Ausführungsform enthält das Reservoir, vorzugsweise das gesamte TTS keines der folgenden Antioxidationsmittel: Tocopherole und dessen Ester, das Sesamol des Sesamöls, das Koniferylbenzoat des Benzoeharzes, die Nordihydroguajakharz- und - guajaretsäure (NDGA), die Gallate (Methyl-, Ethyl-, Propyl-, Amyl-, Butyl-, Lauryl- u.a. Gallate), das Butylhydroxdyanisol (BHT, auch Butyl-p-Kresol genannt); Ascorbinsäure und Salze davon, Ascorbylpalmitat, Erythorbinsäure (iso-Ascorbinsäure) und Salze davon, Monothioglycerol, Natriumformaldehydsulfoxylat, Natriummetabisulfit, Natriumbisulfit, Natriumsulfit, Kaliummetabisulfit, butyliertes Hydroxyanisol, butyliertes Hydroxytoluol, Propionsäure. In einer speziellen Ausführungsform enthält das Reservoir, bevorzugter das gesamte TTS, überhaupt keine Antioxidationsmittel.

Die Menge an Antioxidationsmittel im TTS der vorliegenden Erfindung ist üblicherweise weniger als 1 Gew.-% oder weniger als 0.1 Gew.-%, bevorzugter weniger als 0.05 Gew.-%, am bevorzugtesten weniger als 0.01 Gew.-%, jeweils bezogen auf das Gewicht der Gesamtformulierung (exklusive der Deck- und Abziehschicht).

Der Aufbau des erfindungsgemäßen TTS umfasst mehrere Schichten. Auf dem bei der Anwendung der Haut gegenüber befindlichen Ende des TTS befindet sich die Deckschicht. An der der menschlichen Haut beim Gebrauch zugewandten Seite der Deckschicht befindet sich das Reservoir. Weiterhin befindet sich an der der menschlichen Haut

beim Gebrauch zugewandten Seite des Reservoirs die Klebeschicht. An der der menschlichen Haut beim Gebrauch zugewandten Seite der Klebeschicht befindet sich vor der Anwendung des TTS die Abziehschicht, welche unmittelbar vor dem Gebrauch des TTS entfernt wird.

Die Fläche des erfindungsgemäßen TTS ist nicht besonders eingeschränkt. Üblicherweise beträgt die Fläche etwa 5 - 30 $cm^2$, kann jedoch durchaus größer oder kleiner sein.

Die Fläche der Deckschicht des erfindungsgemäßen TTS entspricht in einer Ausführungsform mindestens der Fläche des Reservoirs beziehungsweise der Klebeschicht. Sie kann jedoch auch größer als die des Reservoirs sein, sodass sie nicht nur das Reservoir vollständig bedeckt, sondern auch über den Rand des Reservoirs hinausragt. In einer solchen Ausführungsform sollte jedoch entweder die Fläche der Klebeschicht gleich der Fläche der Deckschicht sein oder die der Haut zugewandten Seite der Deckschicht eine weitere Klebeschicht aufweisen, um zu gewährleisten, dass die gesamte der Haut zugewandten Oberfläche des TTS bei der Anwendung auf der Haut haftet. In einer anderen Ausführungsform ist die Deckschicht etwas kleiner als die Fläche des Reservoirs.

*Reservoir*

Das Reservoir des erfindungsgemäßen TTS enthält den Wirkstoff Rivastigmin eingebettet in eine Polymermatrix. Die Polymermatrix besteht gemäß diesem Aspekt der Erfindung ausschließlich aus Polymeren oder Copolymeren, die keine Hydroxylgruppen und keine Carboxylgruppen aufweisen. Bevorzugte Polymere oder Copolymere ohne funktionelle Gruppen, die die Polymermatrix bilden, sind bestimmte Polyacrylate, Acrylat-Vinylacetat Copolymere, Polyisobutylen und Styrol-Butadien Copolymere, welche einzeln oder als Mischung vorliegen können.

Als geeignete Polyacrylate, die im wesentlichen keine freien funktionellen Gruppen enthalten, können Polymere (Homopolymere, Copolymere und Block-Copolymere) auf Basis von Acrylsäureestern und/oder Methacrylsäureestern verwendet werden. Als Monomere zur Herstellung geeigneter Polyacrylate kommen dabei insbesondere n-Butylacrylat, n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen dieser Monomere in Frage. Es handelt sich bei diesen Monomeren um Ester der Acryl- bzw. Methacrylsäure, die lineare, verzweigte oder cyclische aliphatische $C_1$-$C_{12}$- Substituenten ohne sonstige freie funktionelle Gruppen tragen. Auch Vinylacetat kann als Co-Monomer zusammen mit mindestens einem dieser Monomere zur Herstellung des Polyacrylats verwendet werden.

Die Polymermatrix besteht vorzugsweise aus einem oder mehreren Polyacrylaten, die im wesentlichen keine freien funktionellen Gruppen enthalten. Bevorzugter besteht die Polymermatrix aus Polyacrylaten, die durch Polymerisierung von Acrylsäureestern und/oder Methacrylsäureestern hergestellt wurden. In einer besonderen Ausführungsform besteht die Polymermatrix aus Polyacrylaten, die durch Polymerisierung von Acrylsäureestern und/oder Methacrylsäureestern hergestellt wurden, wobei die Acrylsäureester und/oder Methacrylsäureester ausgewählt sind aus der Gruppe bestehend aus n-Butylacrylat, n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen davon. In einer anderen Ausführungsform besteht die Polymermatrix im wesentlichen aus Polyacrylaten, die durch Copolymerisierung von Acrylsäureestern und/oder Methacrylsäureestern mit Vinylacetat hergestellt wurden, wobei die Acrylsäureester und/oder Methacrylsäureester ausgewählt sind aus der Gruppe bestehend aus n-Butylacrylat, n-Butylmethacrylat, Ethylacrylat, 2-Ethylhexylacrylat, Ethylmethacrylat, Methylacrylat, Methylmethacrylat, tert-Butylacrylat, sec-Butylacrylat, tert-Butylmethacrylat, Cyclohexylmethacrylat, 2-Ethylhexylmethacrylat, Isobornylmethacrylat, Isobutylmethacrylat, Isopropylacrylat, Isopropylmethacrylat und Mischungen davon.

Besonders bevorzugt sind: das Acrylat-Vinylacetat Copolymer Duro-Tak® 87-4098, welches zu jeweils etwa 50 % aus den Startmonomeren 2-Ethylhexylacrylat und Vinylacetat hergestellt wird, sowie das Acrylat Duro-Tak® 87-9088 (ebenfalls ein AcrylatPolymer ohne funktionelle Gruppen), erhältlich von der Firma Henkel. In einer speziellen Ausführungsform wird für die Polymermatrix das Acrylat Duro-Tak® 87-900A oder Duro-Tak® 87-9301 eingesetzt.

Der Gesamtanteil von Monomeren, die freie Hydroxylgruppen oder freie Carboxylgruppen enthalten (z. B. Acrylsäure, Methacrylsäure und Ester der Acrylsäure bzw. Methacrylsäure, welche funktionelle Gruppen tragen, insbesondere die hydroxylgruppenhaltigen Ester), liegt unterhalb von 1 Gew. -%, vorzugsweise unterhalb von 0,5 Gew. -%, bevorzugter unterhalb von 0,2 ,Gew.-%; bezogen auf das Monomerengemisch, aus der die Polymermatrix hergestellt wird. In einer besonderen Ausführungsform liegt der Gesamtanteil dieser Monomere unterhalb von 0,1 Gew. -%. In einer besonderen Ausführungsform sind keine freien Hydroxylgruppen und keine freien Carboxylgruppen im Monomerengemisch enthalten.

Ein TTS, welches als wirkstoffhaltige Polymermatrix Polyacrylate enthält, die frei von Hydroxylgruppen und Carboxylgruppen sind, wurde zwar in der WO 03/017988 A1 bereits beschrieben, jedoch nicht in Verbindung mit dem Wirkstoff Rivastigmin. Die in der WO 03/017988 beschriebene Aufgabe war es, den Nachteil der geringen Wirkstoffausnutzung eines TTS zu lösen. Gemäß dieser Offenbarung wurde diese Aufgabe durch Polymermatrices gelöst, welche

im Idealfall frei von Hydroxylgruppen oder Carboxylgruppen sind. Der Wirkstoff Rivastigmin wird in dieser Druckschrift nicht erwähnt, geschweige denn eine die Stabilität von Rivastigmin steigernde Wirkung beschrieben.

Das Reservoir enthält in einer Ausführungsform der Erfindung im wesentlichen keine Polymeren oder Copolymeren, die freie Hydroxylgruppen oder freie Carboxylgruppen enthalten. Vorzugsweise enthält das Reservoir im wesentlichen keine freien Hydroxylgruppen und keine freien Carboxylgruppen. Bevorzugter enthält das Reservoir im wesentlichen keine freien Aminogruppen, keine freien Hydroxylgruppen und keine freien Carboxylgruppen. In einer besonderen Ausführungsform enthält auch die Klebeschicht im wesentlichen keine Polymeren oder Copolymeren, die freie Hydroxylgruppen oder freie Carboxylgruppen enthalten. Vorzugsweise enthält die Klebeschicht im wesentlichen keine freien Hydroxylgruppen und keine freien Carboxylgruppen. Bevorzugter enthält die Klebeschicht im wesentlichen keine freien Aminogruppen, keine freien Hydroxylgruppen und keine freien Carboxylgruppen.

Das Reservoir enthält vorzugsweise 20 - 40 Gew.-% Rivastigmin und 60 - 80 Gew.-% Polymermatrix, bezogen auf das Gesamtgewicht des Reservoirs. In einer besonders bevorzugten Ausführungsform des erfindungsgemäßen TTS enthält das Reservoir 25 Gew.-% Rivastigmin und 75 Gew.-% Polymermatrix. Bevorzugt enthält das Reservoir neben dem Wirkstoff und der Polymermatrix keine weiteren Bestandteile. Es ist jedoch möglich, dass zusätzlich weitere im Stand der Technik bekannte Zusatzstoffe im Reservoir enthalten sind. So können beispielsweise Weichmacher oder Gelbildner zusätzlich im Reservoir vorhanden sein.

Die absolute Menge an Rivastigmin hängt von verschiedenen Faktoren, insbesondere von der Größe des einzusetzenden TTS, dem Flächengewicht und der Wirkstoffkonzentration im Reservoir ab. Die Flächengewichte der getrockneten Reservoir Matrix liegen bevorzugt in einem Bereich von 20 - 100 g/m$^2$, weiter bevorzugt in einem Bereich von 25 - 80 g/m$^2$ und noch weiter bevorzugt in einem Bereich von 30 - 70 g/m$^2$. Das Reservoir kann eine Dicke (Trockendicke) im Bereich von 20 - 400 μm, oder 30 - 200 μm oder 40 - 100 μm aufweisen. Auch andere Dicken als die vorstehend genannten sind möglich.

*Klebeschicht*

Die Klebeschicht des erfindungsgemäßen TTS enthält einen Haftkleber, welcher bevorzugt aus Polyisobutylen besteht. Polyisobutylen ist ein selbstklebender Haftkleber, der nicht aushärtet und daher seine klebenden Eigenschaften über einen langen Zeitraum beibehält. Bevorzugterweise werden Polyisobutylene mit unterschiedlichem mittlerem Molekulargewicht als Mischung eingesetzt. Polyisobutylen ist in verschiedenen mittleren Molekulargewichten erhältlich. Der Ausdruck "mittleres Molekulargewicht" im Zusammenhang mit Polyisobutylen bezieht sich in der vorliegenden Anmeldung auf des sog. Viskositätsmittel $M_V$,. Das Viskositätsmittel $M_V$ wird aus der Lösungsviskosität einer Lösung des Polyisobutylens in Isooctan bei 20 °C bestimmt. Als Meßgerät dient ein Ubbelohde-Viskosimeter. Das Viskositätsmittel $M_V$ errechnet sich aus folgender Formel:

$$M_V = 0.65 \sqrt{\frac{J_o \times 10^2}{3.06}}$$

Die Ermittlung des zur Bestimmung des Viskositätsmittels $M_V$ benötigten Staudingerindex $J_o$ erfolgt nach der Schulz-Blaschke-Beziehung aus der gemessenen spezifischen Viskosität $\eta_{SP}$ und der Lösungskonzentration.

$$J_o = \eta_{sp}/c\ (1 + 0.31 \times \eta_{sp})\ cm^3/g \quad \text{(Schulze-Blaschke-Beziehung)}$$

Die spezifische Viskosität $\eta_{SP} = t/t_0 - 1$, wobei t und $t_0$ die Flusszeit der Lösung bzw. des Lösungsmittels (jeweils mit Hagenbach-Couette-Korrektur) sind, und c die Konzentration der Lösung in g/cm$^3$ ist. Gegebenenfalls kann die Vorschrift DIN 53728 ergänzend herangezogen werden.

Geeignete mittlere Molekulargewichte $M_V$ von Polyisobutylen liegen beispielsweise im Bereich von etwa 40.000 g/mol bis etwa 4.000.000 g/mol. Eine bevorzugte Mischung ist die von (1) Polyisobutylen mit einem mittleren Molekulargewicht $M_V$ von etwa 40.000 g/mol (z. B. Oppanol® B10, erhältlich von der Firma BASF) und (2) Polyisobutylen mit einem mittleren Molekulargewicht $M_V$ von über etwa 1.000.000 g/mol (z. B. Oppanol® B100, erhältlich von der Firma BASF, mit einem mittleren Molekulargewicht $M_V$ von etwa 1.110.000 g/mol). Es liegt innerhalb der Kenntnis eines Fachmanns, die verschiedenen Molekulargewichte im geeigneten Verhältnis zu mischen, um die gewünschten Eigenschaften der Klebeschicht zu erzielen.

Das Polyisobutylen in der Klebeschicht kann eine Molekulargewichtsverteilung aufweisen, die ein erstes relatives Maximum zwischen 30.000 g/mol und 100.000 g/mol, und ein zweites relatives Maximum zwischen 300.000 g/mol

und 500.000 g/mol aufweist. Bevorzugter liegt das erste relative Maximum zwischen 35.000 g/mol und 50.000 g/mol und, unabhängig davon, dass zweite relative Maximum zwischen 350.000 g/mol und 450.000 g/mol. Am bevorzugtesten liegt das erste relative Maximum bei etwa 40.000 g/mol, und, unabhängig davon, das zweite relative Maximum bei etwa 400.000 g/mol.

Das Polyisobutylengemisch des Haftklebers kann dadurch erhalten werden, dass man ein erstes Polyisobutylenpolymer mit einem mittleren Molekulargewicht $M_V$ zwischen 30.000 g/mol und 100.000 g/mol mit einem zweiten Polyisobutylenpolymer mit einem mittleren Molekulargewicht $M_V$ zwischen 300.000 g/mol und 500.000 g/mol mischt. Das erste Polyisobutylenpolymer hat vorzugsweise ein mittleres Molekulargewicht $M_V$ zwischen 35.000 g/mol und 50.000 g/mol, am bevorzugtesten von etwa 40.000 g/mol. Das zweite Polyisobutylenpolymer hat vorzugsweise ein mittleres Molekulargewicht $M_V$ zwischen 350.000 g/mol und 450.000 g/mol, am bevorzugtesten von etwa 400.000 g/mol.

Die bevorzugteste Mischung ist die von (1) Polyisobutylen mit einem mittleren Molekulargewicht $M_V$ von etwa 40.000 g/mol (zum Beispiel Oppanol® B10 SFN, erhältlich von der Firma BASF) und (2) Polyisobutylen mit einem mittleren Molekulargewicht $M_V$ von etwa 400.000 g/mol (zum Beispiel Oppanol® B50 SF, erhältlich von der Firma BASF).

Die Anteile der beiden Polyisobutylenpolymere in dem Gemisch können variieren. Das Gewichtsverhältnis des ersten Polyisobutylenpolymer zum zweiten Polyisobutylenpolymer in dem Gemisch kann 10:1 bis 1:10 betragen, vorzugsweise 2:1 bis 1:2, am bevorzugtesten ist das Verhältnis etwa 4:6. In einer besonders bevorzugten Ausführungsform besteht das Polyisobutylenpolymer des Haftklebers aus 4 Gewichtsteilen Oppanol® B10 (zum Beispiel Oppanol® B10 SFN) und 6 Gewichtsteilen Oppanol® B50SF.

Der Haftkleber ist in der Klebeschicht erfindungsgemäß in einer Menge im Bereich von 40 - 100 Gew.-%, bevorzugt 50 - 90 Gew.-%, weiter bevorzugt 55 - 80 Gew.-% und noch weiter bevorzugt 60 - 69,9 Gew.-%, bezogen auf das Gesamtgewicht der Klebeschicht, vorhanden.

Die Dicke der Klebeschicht (Trockendicke) ist nicht besonders eingeschränkt. Sie kann in einem Bereich von etwa 10 - 300 µm, oder in einem Bereich von 70 -140 µm liegen. Die absolute Menge der Klebeschicht kann etwa 10 - 50 g/m$^2$, oder 20 - 40 g/m$^2$ betragen, ohne darauf eingeschränkt zu sein.

Die Klebeschicht enthält bevorzugt zusätzlich Weichmacher und Gelbildner. Geeignete Weichmacher sind im Stand der Technik bekannt, wobei es sich bevorzugt um Mineralöl, Neutralöl, Paraffin, Polybutene, Leinsamenöl, Octylpalmitat, Squalen, Squalan, Silikonöl, Isobutylmyristat, Isostearylalkohol und/oder Oleylalkohol, besonders bevorzugt um Mineralöl, Neutralöl, Polybutene und/oder Paraffin handelt. Mineralöle sind farblose, klare Kohlenwasserstoffe. Sie werden aus den oberhalb von etwa 300°C siedenden Destillationsfraktionen des Erdöls gewonnen und durch Abkühlen von festen Kohlenwasserstoffen befreit. Durch geeignete Fraktionierung können Mineralöle gewonnen werden, die etwa bei Körpertemperatur, also bei etwa 35 - 37°C flüssig sind und bei tiefen Temperaturen, insbesondere bei Temperaturen unter 20°C fest sind. Die Auswahl des Mineralöls mit einem Verflüssigungspunkt von etwa 30 - 35°C ist bevorzugt. Besonders bevorzugt sind Paraffine und Mineralöle, welche den Anforderungen der Ph. Eur. 6 und/oder der USP 32-NF 27 entsprechen.

Der Weichmacher ist in der Klebeschicht bevorzugt in einer Menge im Bereich von 0 - 60 Gew.-%, weiter bevorzugt von 10 - 50 Gew.-%, noch weiter bevorzugt im Bereich von 25 - 39,9 Gew.-%, beispielsweise 35 Gew.-% bezogen auf das Gesamtgewicht der Klebeschicht, vorhanden.

Bei dem Gelbildner handelt es sich bevorzugt um einen Gelbildner mit partikulärer Struktur, welcher auf seiner Oberfläche eine hohe Konzentration polarer Gruppen aufweist. Diese verursachen zu den Ölen hin entsprechend hohe Grenzflächenspannungen, die durch Agglomeration der Partikel untereinander zu Gelgerüsten teilweise kompensiert werden. Demzufolge sind die Gelgerüste immer umso fester, je größer der Polaritätsunterschied zwischen den Ölen und der Gerüstbildneroberfläche ist. Bevorzugt wird erfindungsgemäß als Gelbildner hochdisperes Siliziumdioxid oder pyrogene Kieselsäure eingesetzt. Die Größe der Partikel bewegt sich bevorzugt im Nanometerbereich und liegt zum Beispiel im Bereich von 400 - 1500 nm, insbesondere im Bereich von 500 - 1000 nm. Pyrogene Kieselsäure wird beispielsweise unter der Bezeichnung CAB-O-SIL® vertrieben und ist ein bekanntes Verdickungsmittel für Mineralöl. Ein anderes Beispiel für einen geeigneten Gelbildner ist Bentonit. Auch das als Gelbildner bekannte Natriumcarbomer kann verwendet werden. Eingesetzt wird der Gelbildner bevorzugt in einer Menge von 0,1 - 4,0 Gew.-%, weiter bevorzugt 0,1 - 2,0 Gew.-%, noch weiter bevorzugt 0,5 - 2,0 Gew.-%, bezogen auf das Gewicht der Klebeschicht.

In einer besonders bevorzugten Ausführungsform des erfindungsgemäßen TTS enthält die Klebeschicht 60 - 69,9 Gew.-% Haftkleber, beziehungsweise Polyisobutylen, wobei dieses wie oben beschrieben aus Mischungen von Polyisobutylen mit unterschiedlichen Molekulargewichten bestehen kann, 0,1 - 2,0 Gew.-% Gelbildner, beziehungsweise hochdisperses Siliziumdioxid oder pyrogene Kieselsäure, und 25 - 39,9 Gew.-% Weichmacher, beziehungsweise Mineralöl, bezogen auf das Gesamtgewicht der Klebeschicht.

Die Deckschicht des erfindungsgemäßen TTS ist vorzugsweise okklusiv, also abschließend. Derartige Deckschichten können in bevorzugter Ausführungsform aus Polyolefinen, insbesondere Polyethylen, oder aus Polyester sowie Polyurethanen bestehen. Auch Schichten, die mehrere verschiedene Polymere übereinander angeordnet beinhalten, sind vorteilhafterweise einsetzbar. Geeignete Materialien umfassen Polyolefin, Zellophan, Celluloseacetat, Ethylcellulose, mit Weichmachern versehene Vinylacetat-Vinylchlorid Copolymere, Ethylen-Vinylacet Copolymere, Polyethylentherephtalat, Nylon, Polyethylen, Polypropylen, Polyvinylidenchlorid, Ethylen-Metacrylat Copolymere, Papier, das gegebenenfalls beschichtet sein kann, textile Gewebe, Aluminiumfolie und Polymer-Metall Kompositmaterialien. Besonders bevorzugt sind Polyesterfolien, wie Polyethylentherephtalatfolien. Die Dicke der Rückschicht kann, wie im Stand der Technik üblich, zum Beispiel 10 µm bis 100 µm, zum Beispiel etwa 40 µm (nominelle Dicke) betragen. Ganz besonders bevorzugt sind Verbandfolien aus pigmentiertem PE, PETP und Aluminium

Auf der Klebeschicht befindet sich erfindungsgemäß eine Abziehschicht, auch als "release liner" bezeichnet. Diese Abziehschicht ist bevorzugt aus polymerem Material hergestellt, das gegebenenfalls auch metallisiert sein kann. Beispiele für bevorzugt eingesetzte Materialien sind Polyurethane, Polyvinylacetat, Polyvinylidenchlorid, Polypropylen, Polycarbonat, Polystyrol, Polyethylen, Polyethylentherephtalat, Polybutylentherephtalat sowie gegebenenfalls mit entsprechenden Polymeren oberflächenbeschichtetes Papier. Bevorzugt handelt es sich hierbei um eine einseitig oder beidseitig fluoropolymerbeschichtete oder silikonisierte Abziehschicht. Besonders bevorzugt sind handelsübliche fluoropolymerbeschichtete oder silikonisierte Polyesterfolien, wie die einseitig silikonisierten Handelsprodukte Primeliner 100 µm und Perlasic LF 75 µm (Firma Loparex, NL und Perlen Converting AG, Schweiz) oder die einseitig fluoropolymerbeschichtete Produkte wie z.B. ScotchPak 1022 (3M Drug delivery).

Eine besonders bevorzugte Ausführungsform des erfindungsgemäßen TTS ist ein TTS zur Verabreichung eines Wirkstoffes durch die Haut, umfassend:

a) eine Deckschicht,

b) ein auf der Deckschicht befindliches Reservoir enthaltend 20 - 30 Gew.-% Wirkstoff und 70 - 80 % Polymermatrix, bezogen auf das Gesamtgewicht des Reservoirs, wobei die Polymermatrix im wesentlichen aus einem Acrylat-Vinylacetat-Copolymer ohne Hydroxylgruppen und ohne Carboxylgruppen besteht, und wobei der Wirkstoff Rivastigmin oder ein physiologisch verträgliches Salz, Hydrat oder Solvat davon ist;

c) eine auf dem Reservoir befindliche Klebeschicht umfassend 0,1 - 1 Gew.-% Siliziumdioxid, 25 - 39.9 Gew.-% leichtes Mineralöl (Paraffinum perliquidum) und als Haftkleber 60 - 79,9 Gew.-% eines Gemisches aus einem Polyisobutylenpolymer mit einem mittleren Molekulargewicht $M_V$ von etwa 40.000 g/mol und einem Polyisobutylenpolymer mit einem mittleren Molekulargewicht $M_V$ von etwa 400.000 g/mol; und

d) eine auf der Klebeschicht befindliche Abziehschicht,

wobei die Menge an Antioxidationsmittel in der Gesamtformulierung ohne Deck- und Abziehschicht weniger als 0,1 Gew.-%, bevorzugt weniger als 0,01% ist, und das Reservoir kein Tocopherol, kein Butylhydroxdyanisol und kein Butylhydroxytoluol enthält.

In einer besonderen Ausführungsform enthält das erfindungsgemäße TTS kein Antioxidationsmittel ausgewählt aus der Gruppe bestehend aus Vitamin E und Estern davon, Butyhydroxytoluol und Butylhydroxyanisol.

Ein weiterer Aspekt der vorliegenden Erfindung ist die Bereitstellung eines Verfahrens zur Herstellung des erfindungsgemäßen TTS. Das Verfahren umfasst

i) die Herstellung einer das Reservoir enthaltenden Komponente, enthaltend die Deckschicht und das Reservoir, welches sich auf der Seite der Deckschicht befindet, welche als die der Haut zugewandten Seite bestimmt ist,

ii) die Herstellung einer die Klebeschicht enthaltenden Komponente, enthaltend die Abziehschicht und die sich auf der Abziehschicht befindliche Klebeschicht und

iii) das aufeinander Laminieren der Komponenten aus i) und ii), so dass Deck- und Abziehschicht im Querschnitt des fertigen TTS die gegenüberliegenden, äußersten Schichten darstellen.

Eine bevorzugte Ausführungsform dieses Verfahrens umfasst

i) das Auftragen und gegebenenfalls anschließende Trocknen eines Films einer das Reservoir bildenden Zusammensetzung, gegebenenfalls in Form einer Lösung oder Dispersion in einem geeigneten Medium, auf die Seite der Deckschicht, welche als die der Haut zugewandte Seite bestimmt ist, und gegebenenfalls das Kaschieren mit einer silikonisierten Abziehfolie (Liner); oder das Aufbringen und gegebenenfalls anschließende Trocknen eines Films einer das Reservoir bildenden Zusammensetzung, gegebenenfalls in Form einer Lösung oder Dispersion in einem geeigneten Medium, auf eine silikonisierte Abziehfolie (Liner) und das Kaschieren mit einer Deckschicht;

ii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf die Abziehschicht und

iii) das aufeinander Laminieren der Komponenten aus i) und ii), gegebenenfalls unter Abziehen von Liner, so dass Deck- und Abziehschicht im Querschnitt des fertigen TTS die gegenüberliegenden, äußersten Schichten darstellen.

Die Herstellung eines bevorzugten TTS kann erfolgen, indem man zunächst die Komponenten für das Reservoir, also Rivastigmin und das matrixbildende Polymer, bzw. Copolymer oder eine Mischung davon, in einem organischen Lösungsmittel wie Heptan oder Ethylacetat dispergiert, bzw. löst (sofern das Polymer nicht bereits gelöst vorliegt). Üblicherweise liegt das matrixbildende Polymer, bzw. Copolymer oder die Mischung davon bereits in einem Lösungsmittel vor. Dabei wird erfindungsgemäß ein Polymer und/oder Copolymer eingesetzt, das wie oben im Zusammenhang mit dem erfindungsgemäßen TTS definiert ist, also ein Polymer und/oder Copolymer ohne Hydroxylgruppen und ohne Carboxylgruppen. Die oben als bevorzugt genannten Ausführungsformen der Polymermatrix gelten entsprechend für das erfindungsgemäße Verfahren. Bei der Herstellung des Reservoirs wird bevorzugt ein flüchtiges organisches Lösungsmittel eingesetzt. Diese Mischung wird dann als gleichmäßige Schicht auf der Deckschicht aufgebracht und getrocknet. Vorzugsweise wird die Komponente für das Reservoir zum Schutz mit einer Folie, bevorzugt eine silikonisierte Polyesterfolie, auch als "intermediate liner" bezeichnet, versehen, welche auf der der Deckschicht gegenüberliegenden Seite des Reservoirs aufgebracht wird. Alternativ, bzw. hierzu äquivalent kann die Mischung auch zunächst auf den "intermediate liner" aufgetragen und getrocknet werden, wobei die Deckschicht im Anschluss auf die dem "intermediate liner" gegenüberliegenden Seite des Reservoirs aufgebracht wird. Der "intermediate liner" wird entfernt, kurz bevor die Reservoirkomponente mit der die Klebeschicht enthaltenden Komponente zusammengefügt wird.

In einem separaten Arbeitsschritt wird die Klebeschicht hergestellt, indem das den Haftkleber bildende (in organischem Lösungsmittel gelöste) Polymerengemisch, bevorzugt Polyisobutylen von unterschiedlichem mittleren Molekulargewicht, zusammen mit dem Gelbildner und dem Weichmacher in einem organischen Lösungsmittel wie Heptan dispergiert wird. Bevorzugt wird jedoch der Haftkleber und der Weichmacher in dem organischen Lösungsmittel gelöst und anschließend der Gelbildner in dieser Lösung dispergiert. Diese Mischung wird dann auf die Abziehfolie aufgebracht und trocknen gelassen.

Die in den beiden Verfahrensschritten erhaltenen Komponenten werden anschließend miteinander laminiert, und zwar so, dass die Klebeschicht direkt auf das Reservoir aufgebracht wird. Anschließend können aus der fertigen laminierten Folie Stücke gewünschter Größe ausgestanzt und verpackt werden.

Bei den einzelnen Verfahrensschritten werden die organischen Lösungsmittel, die erforderlich sind, um die jeweiligen Komponenten in Lösung zu bringen bzw. zu dispergieren, dadurch entfernt, dass die Produkte ansteigenden Temperaturen, gegebenenfalls auch unter Anwendung eines Unterdrucks, ausgesetzt werden.

Einen weiteren Aspekt der vorliegenden Erfindung stellt die Verwendung eines Polymers oder Copolymers, das weder Hydroxylgruppen noch Carboxylgruppen aufweist, in einem TTS enthaltend Rivastigmin dar. Bevorzugt ist dabei die Verwendung von Polyacrylaten, Acrylat-Vinylacetat Copolymeren, Polyisobutylen und Styrol-Butadien Copolymeren. Erfindungsgemäß stellen diese Polymere oder Copolymere die Polymermatrix des Reservoirs dar, in die der Wirkstoff Rivastigmin eingebettet ist.

In der erfindungsgemäßen Verwendung kommen bevorzugt hydroxylgruppenfreie und carboxylgruppenfreie Polyacrylate und Polyacrylatcopolymere, wie Acrylat-Vinylacetat Copolymere zum Einsatz.

In einer besonders bevorzugten Ausführungsform der erfindungsgemäßen Verwendung wir das Acrylat-Vinylacetat Copolymer Duro-Tak® 87-4098 verwendet.

Ein weiterer Aspekt der vorliegenden Erfindung ist die Bereitstellung des erfindungsgemäßen TTS zur Behandlung von Alzheimer und Parkinsondemenz. Das erfindungsgemäße TTS wird dabei bevorzugt für einen Anwendungszeitraum von ungefähr 24 Stunden hergerichtet.

Im Folgenden werden bevorzugte Ausführungsformen des erfindungsgemäßen TTS anhand von experimentellen Beispielen beschrieben und deren Eigenschaften hinsichtlich der Stabilität bestimmt.

## Beispiele

### Beispiel 1

Herstellung verschiedener Formulierungen

Es wurden vier unterschiedliche Chargen A-D von TTS-Formulierungen, welche Rivastigmin Base enthalten, hergestellt. Für das Reservoir wurde Acrylatvinylacetatcopolymer als matrixbildendes Polymer verwendet. Eine Übersicht der Bestandteile der verschiedenen Chargen ist in Tabelle 1 wiedergegeben.

Tabelle 1: Zusammensetzung der Chargen A bis D

| **Charge** | Zusammensetzung | |
|---|---|---|
| A | | 30% Rivastigmin Base<br>70% Duro-Tak® 87-4098 |
| B | Reservoir:<br><br>Klebeschicht: | 30% Rivastigmin Base<br>70% Duro-Tak® 87-4098<br>30% Klearol®<br>1% Cab-O-Sil®<br>69% Oppanol® B10/B100 (7/3) |
| C | Reservoir:<br><br>Klebeschicht: | 20% Rivastigmin Base<br>80% Duro-Tak® 87-4098<br>30% Klearol®<br>0,5% Cab-O-Sil®<br>69,5% Oppanol® B10/B100 (7/3) |
| D | Reservoir:<br><br>Klebeschicht: | 25% Rivastigmin Base<br>75% Duro-Tak® 87-4098<br>30% Klearol®<br>1% Cab-O-Sil®<br>69% Oppanol® B10/B100 (7/3) |

Die in den Chargen eingesetzten Komponenten können folgendermaßen näher beschrieben werden:

Tabelle 2: Übersicht der Komponenten der Formulierungsbeispiele A bis D

| **Komponentenbezeichnung** | **Chemische Beschreibung** | **Funktion** |
|---|---|---|
| Duro-Tak® 87-4098 | Acrylat/Vinylacetat Copolymer | Matrixpolymer |
| Cab-O-Sil® | Pyrogenes Siliziumdioxid | Gelbildner |
| Klearol® | Leichtes Mineralöl | Weichmacher |
| Oppanol® B10 | Polyisobutylen ($M_w$= 4x$10^4$ g/mol) | Haftkleber |
| Oppanol® B100 | Polyisobutylen ($M_w$= 1,1x$10^6$ g/mol) | Haftkleber |

Herstellungsverfahren

1. Herstellung der Reservoirschicht

Der Acrylat-Vinylacetat Kleber wird vorgelegt und Rivastigmin und Ethylacetat werden dazu gewogen. Anschliessend werden die Komponenten in ausreichend Ethylacetat mittels eines Rührers gemischt, sodass eine streichfähige, homogene Beschichtungsmasse entsteht.

Die homogene Beschichtungsmasse wird auf einer silikonisierten Folie ("intermediate liner") als dünner Film aufgetragen. Der Matrixfilm wird bei 60°C/20 min und 80°C/5 min getrocknet und anschliessend mit einer Deckschicht aus PET kaschiert.

2. Herstellung der Adhesivschicht und des Gesamtlaminats

Die Polyisobutylenkleber werden zusammen eingewogen und gemischt. Anschliessend werden unter Rühren Heptan, Klearol® und Cab-O-Sil® zugegeben und so lange gerührt bis die Masse homogen ist.

Die Masse wird auf eine Abziehschicht ("release liner") als dünner Film aufgetragen und anschliessend werden die Lösemittel bei 60°C/20 min und 80°C/5 min abgezogen. Nach der Trocknung wird das Laminat mit der Reservoirschicht kaschiert, wobei der "intermediate liner" vorher entfernt wird.

Aus dem erhaltenen Laminat werden Pflaster geeigneter Grösse ausgestanzt.

Stabilitätstests

Die Chargen A und B wurden Stabilitätstests unterzogen. Dabei wurden die ausgestanzten TTS in Aluminiumfoliebeutel versiegelt und jeweils einen Monat lang bei 25°C und 60 % relativer Luftfeuchtigkeit, beziehungsweise bei 40°C und 75 % relativer Luftfeuchtigkeit gelagert. Anschließend wurde der Gehalt eventuell entstandener Verunreinigungen als Folge der Zersetzung von Rivastigmin mittels HPLC und UV-Absorption ermittelt. Die Ergebnisse dieser Versuche sind in Tabelle 3 zusammengefasst.

Tabelle 3

| Charge | Verunreinigungen | | Lagerdauer |
|---|---|---|---|
| | **25 °C / 60% r.F.** | **40°C / 75% r.F.** | |
| A | RT = 46 min: 0,18%<br>Total: 0,18% | --- | Initial |
| | RT = 46 min: 0,22%<br>Total: **0,22%** | RT = 46 min: 0,29%<br>**Total: 0,29%** | 1 Monat |
| B | RT = 46 min: 0,34%<br>**Total: 0,34%** | RT = 46 min: 0,35%<br>Total: **0,35%** | 2 Wochen |
| | RT = 46 min: 0,36%<br>**Total: 0,36%** | RT = 46 min: 0,38%<br>Total: **0,38%** | 1 Monat |

Die festgestellte Verunreinigung ist mit ihrer jeweiligen Retentionszeit (RT) angegeben. Die prozentuale Mengenangabe der Verunreinigung stellt den Anteil am Gesamtgewicht der betreffenden Formulierungen dar. Die Untergrenze der angegebenen Messwerte RL (Reporting Limit) beträgt 0,1 %, da sich darunterliegende Werte im Bereich der Messungenauigkeit befinden.

Die Ergebnisse dieser Studie zeigen deutlich, dass die erfindungsgemäßen TTS Formulierungen hinreichend stabil sind. Bei allen Formulierungsbeispielen liegt die Verunreinigung RT=46 min nach einem Monat bei 40°C und 75 % relative Luftfeuchtigkeit bei unter 0,4 %.

**Beispiel 2**

Herstellung verschiedener Formulierungen

Es wurden zwei unterschiedliche Chargen von TTS-Formulierungen, welche Rivastigminbase enthalten, hergestellt. Für das Reservoir wurde Acrylatvinylacetatcopolymer als matrixbildendes Polymer verwendet. Eine Übersicht der Bestandteile der verschiedenen Chargen ist in Tabelle 4 wiedergegeben.

Tabelle 4: Zusammensetzung der Chargen 179/181 und 179/182:

| Charge | Zusammensetzung | |
|---|---|---|
| 179/181 | Reservoir: | 25 % Rivastigmin Base<br>75 % Duro-Tak® 87-4098 |
| | Klebeschicht: | 30 % Pionier® 7028P (Hansen & Rosenthal)<br>0,5 % Cab-O-Sil®<br>69,5 % Oppanol® B10 SFN / B50 SF (4/6) |
| 179/182 | Reservoir: | 25 % Rivastigmin Base<br>75 % Duro-Tak® 87-4098 |
| | Klebeschicht: | 35 % Paraffin, dünnfl. (Merck)<br>0,5 % Cab-O-Sil®<br>64,5 % Oppanol® B10 SFN / B50 SF (4/6) |

Die in den Chargen eingesetzten Komponenten können folgendermaßen näher beschrieben werden:

Tabelle 5: Übersicht der Komponenten der Formulierungsbeispiele

| Komponentenbezeichnung | Chemische Beschreibung | Funktion |
|---|---|---|
| Duro-Tak® 87-4098 | Acrylat/Vinylacetat Copolymer | Matrixpolymer |
| Cab-O-Sil® | Pyrogenes Siliziumdioxid | Gelbildner |
| Pionier® 7028P | Leichtes Mineralöl (Paraffinum perliquidum) | Weichmacher |
| Oppanol® B10 SFN | Polyisobutylen ($M_V$= ca. $4x10^4$ g/mol) | Haftkleber |
| Oppanol® B50 SF | Polyisobutylen ($M_V$= ca. $4x10^5$ g/mol) | Haftkleber |

Herstellungsverfahren

1. Herstellung der Reservoirschicht

Der Acrylat-Vinylacetat Kleber wird vorgelegt und Rivastigmin und Ethylacetat werden dazu gewogen. Anschliessend werden die Komponenten in ausreichend Ethylacetat mittels eines Rührers gemischt, sodass eine streichfähige, homogene Beschichtungsmasse entsteht.

Die homogene Beschichtungsmasse wird auf einer silikonisierten Folie ("intermediate liner") als dünner Film aufgetragen. Der Matrixfilm wird bei 60°C/20 min und 80°C/5 min getrocknet und anschliessend mit einer Deckschicht aus PET kaschiert.

2. Herstellung der Adhesivschicht und des Gesamtlaminats

Die Polyisobutylenkleber werden zusammen eingewogen und gemischt. Anschliessend werden unter Rühren Heptan, Pionier® 7028P / Paraffin, dünnfl. und Cab-O-Sil® zugegeben und so lange gerührt bis die Masse homogen ist.

Die Masse wird auf eine Abziehschicht ("release liner") als dünner Film aufgetragen und anschliessend werden die Lösemittel bei 60°C/20 min und 80°C/5 min abgezogen. Nach der Trocknung wird das Laminat mit der Reservoirschicht kaschiert, wobei der "intermediate liner" vorher entfernt wird.

Aus dem erhaltenen Laminat werden Pflaster geeigneter Grösse ausgestanzt.

Stabilitätstests

Die beiden Chargen wurden Stabilitätstests unterzogen. Dabei wurden die ausgestanzten TTS in Beutel aus Aluminium-Verbundfolien versiegelt und jeweils einen Monat lang bei 25°C und 60 % relativer Luftfeuchtigkeit, beziehungsweise bei 40°C und 75 % relativer Luftfeuchtigkeit gelagert. Anschließend wurde der Gehalt eventuell entstandener Verunreinigungen als Folge der Zersetzung von Rivastigmin mittels HPLC und UV-Absorption ermittelt. Die Ergebnisse dieser Versuche sind in den Tabellen 6 und 7 zusammengefasst.

**Tabelle 6: Verunreinigungen in Formulierung 179/181**

| **Initial** | 1 **Monat** | | **3 Monate** | | | **6 Monate** | |
|---|---|---|---|---|---|---|---|
| | **25 °C** | **40 °C** | **5 °C** | **25 °C** | **40 °C** | **25 °C** | **40 °C** |
| Imp 1: < RL<br>Imp 4: < RL<br>Imp 5: < RL<br><br>**Total: < RL** | Imp 1: < RL<br>Imp 4: < RL<br>Imp 5: 0,11%<br><br>**Total: 0,11%** | Imp 1: < RL<br>Imp 4: 0,13%<br>Imp 5: 0,14%<br><br>**Total: 0,27%** | Imp 1: < RL<br>Imp 4: 0,12%<br>Imp 5: 0,17%<br><br>**Total: 0,29%** | Imp 1: < RL<br>Imp 4: 0,12%<br>Imp 5: 0,17%<br><br>**Total: 0,29%** | Imp 1: < RL<br>Imp 4: 0,25%<br>Imp 5: 0,26%<br><br>**Total: 0,51%** | Imp 1: < RL<br>Imp 4: 0,14%<br>Imp 5: 0,18%<br><br>**Total: 0,32%** | Imp 1: < RL<br>Imp 4: 0,30%<br>Imp 5: 0,36%<br><br>**Total: 0,66%** |

**Tabelle 7: Verunreinigungen in Formulierung 179/182**

| Initial | 1 Monat | | 3 Monate | | | 6 Monate | |
|---|---|---|---|---|---|---|---|
| | **25 °C** | **40 °C** | **5 °C** | **25 °C** | **40 °C** | **25 °C** | **40 °C** |
| Imp 1: < RL | Imp 1: < RL | Imp 1: < RL | Imp 1: < RL | Imp 1: < RL | Imp 1: < RL | Imp 1: < RL | Imp 1: < RL |
| Imp 4: < RL | Imp 4: < RL | Imp 4: 0,16% | Imp 4: < RL | Imp 4: 0,13% | Imp 4: 0,25% | Imp 4: 0,14% | Imp 4: 0,36% |
| Imp 5: < RL | Imp 5: 0,11% | Imp 5: 0,17% | Imp 5: 0,13% | Imp 5: 0,15% | Imp 5: 0,28% | Imp 5: 0,18% | Imp 5: 0,41% |
| **Total: < RL** | **Total: 0,11%** | **Total: 0,33%** | **Total: 0,13%** | **Total: 0,28%** | **Total: 0,53%** | **Total: 0,32%** | **Total: 0,77%** |

Die prozentuale Mengenangabe der Verunreinigung stellt den Anteil am Sollgehalt an Wirkstoff in den betreffenden Formulierungen dar. Die Untergrenze der Messwerte (Reporting Limit) der einzelnen Verunreinigungen beträgt 0,1 %, da sich darunterliegende Werte im Bereich der Messungenauigkeit befinden. Neben den in den Tabellen 6 und 7 angegebenen Verunreinigungen wurden keine Verunreinigungen oberhalb des "Reporting Limit" von 0,1% beobachtet.

Die Ergebnisse dieser Studie zeigen deutlich, dass die erfindungsgemäßen TTS Formulierungen hinreichend stabil sind.

Die vorliegende Erfindung betrifft unter anderem folgende Gegenstände (1) bis (19):

(1) Transdermales therapeutisches System zur Verabreichung eines Wirkstoffs durch die Haut umfassend:

a) eine Deckschicht,

b) ein auf der Deckschicht befindliches Reservoir umfassend eine den Wirkstoff enthaltende Polymermatrix, wobei der Wirkstoff Rivastigmin oder ein physiologisch verträgliches Salz, Hydrat oder Solvat davon ist,

c) eine auf dem Reservoir befindliche Klebeschicht umfassend als Haftkleber ein erstes Polyisobutylenpolymer, mit einem mittleren Molekulargewicht $M_V$ zwischen 30.000 g/mol und 100.000 g/mol, und ein zweites Polyisobutylenpolymer, mit einem mittleren Molekulargewicht $M_V$ zwischen 300.000 g/mol und 500.000 g/mol; und

d) eine auf der Klebeschicht befindliche Abziehschicht,

dadurch gekennzeichnet, dass die Polymermatrix des Reservoirs weder Hydroxylgruppen noch Carboxylgruppen aufweist und das Reservoir kein Tocopherol, kein Butylhydroxdyanisol und kein Butylhydroxytoluol enthält.

(2) Transdermales therapeutisches System nach Gegenstand (1), dadurch gekennzeichnet, dass es keine Antioxidationsmittel, ausgewählt aus der Gruppe bestehend aus Tocopherolen und dessen Estern, Sesamol, dem Koniferylbenzoat des Benzoeharzes, Nordihydroguajakharz- und -guajaretsäure, Gallaten, Butylhydroxdyanisol, Ascorbinsäure und Salzen davon, Ascorbylpalmitat, Erythorbinsäure und Salzen davon, Monothioglycerol, Natriumformaldehydsulfoxylat, Natriummetabisulfit, Natriumbisulfit, Natriumsulfit, Kaliummetabisulfit, butyliertem Hydroxyanisol, butyliertem Hydroxytoluol und Propionsäure, enthält.

(3) Transdermales therapeutisches System nach Gegenstand (1) oder (2), wobei das Reservoir 20 - 30 Gew.-% Wirkstoff und 70 - 80 Gew.-% Polymermatrix, bezogen auf das Gesamtgewicht des Reservoirs, enthält.

(4) Transdermales therapeutisches System nach einem der Gegenstände (1) bis (3), wobei die Klebeschicht zusätzlich einen Gelbildner, ausgewählt aus der Gruppe bestehend aus hochdispersem Siliziumdioxid und pyrogener Kieselsäure, und einen Weichmacher, ausgewählt aus der Gruppe bestehend aus Mineralöl, Neutralöl, Paraffin, Polybuten, Leinsamenöl, Octylpalmitat, Squalen, Squalan, Silikonöl, Isobutylmyristat, Isostearylalkohol und Oleylalkohol, enthält.

(5) Transdermales therapeutisches System nach Gegenstand (4), wobei die Klebeschicht 60,0 - 74,9 Gew.-% Haftkleber, 0,1 - 2,0 Gew.-% Gelbildner und 25 - 39,9 Gew.-% Weichmacher, bezogen auf das Gesamtgewicht der Klebeschicht, enthält.

(6) Transdermales therapeutisches System nach einem der Gegenstände (1) bis (5), wobei die Polymermatrix wenigstens ein Polymer und/oder Copolymer umfasst, ausgewählt aus der Gruppe bestehend aus Polyacrylaten, Acrylat-Vinylacetat Copolymeren, Polyisobutylen, Styrol-Butadien Copolymeren und Mischungen davon.

(7) Transdermales therapeutisches System nach einem der Gegenstände (1) bis (6), dadurch gekennzeichnet, dass der Haftkleber Polyisobutylen ist.

(8) Transdermales therapeutisches System nach einem der Gegenstände (1) bis (7), dadurch gekennzeichnet, dass das Polyisobutylen ein Gemisch aus zwei Polyisobutylenpolymeren mit unterschiedlichen Molekulargewichten ist.

(9) Transdermales therapeutisches System nach Gegenstand (8), dadurch gekennzeichnet, dass das erste Polyisobutylenpolymer ein mittleres Molekulargewicht $M_V$ von 40.000 g/mol hat, und das zweite Polyisobutylenpolymer ein mittleres Molekulargewicht $M_V$ von 400.000 g/mol hat.

(10) Transdermales therapeutisches System nach einem der Gegenstände (8) bis (9), dadurch gekennzeichnet, dass das Gewichtsverhältnis des ersten Polyisobutylenpolymers zum zweiten Polyisobutylenpolymer 4:6 ist.

(11) Transdermales therapeutisches System nach einem der Gegenstände (4) bis (10), dadurch gekennzeichnet, dass der Weichmacher Paraffin, Neutralöl, Mineralöl, oder Polybuten oder eine Mischung davon ist.

(12) Transdermales therapeutisches System nach einem der Gegenstände (1) bis (11), wobei die Fläche der Klebeschicht der Fläche des Reservoirs entspricht.

(13) Transdermales therapeutisches System nach Gegenstand (1), umfassend:

a) eine Deckschicht,

b) ein auf der Deckschicht befindliches Reservoir enthaltend 20 - 30 Gew.-% Wirkstoff und 70 - 80 % Polymermatrix, bezogen auf das Gesamtgewicht des Reservoirs, wobei die Polymermatrix im wesentlichen aus einem Acrylat-Vinylacetat-Copolymer ohne Hydroxylgruppen und ohne Carboxylgruppen besteht;

c) eine auf dem Reservoir befindliche Klebeschicht umfassend 0,1 - 1 Gew.-% Siliziumdioxid, 25 - 39.9 Gew.-% Paraffinum perliquidum (Ph. Eur.) und 60 - 79,9 Gew.-% eines Gemisches aus Polyisobutylen mit einem mittleren Molekulargewicht $M_V$ von etwa 40.000 g/mol und Polyisobutylen mit einem mittleren Molekulargewicht $M_V$ von etwa 400.000 g/mol; und

d) eine auf der Klebeschicht befindliche Abziehschicht.

(14) Verfahren zur Herstellung eines transdermalen therapeutischen Systems nach einem der Gegenstände (1) bis (13), umfassend

i) die Herstellung einer das Reservoir enthaltenden Komponente, enthaltend die Deckschicht und das Reservoir, welches sich auf der Seite der Deckschicht befindet, welche als die der Haut zugewandten Seite bestimmt ist,

ii) die Herstellung einer die Klebeschicht enthaltenden Komponente, enthaltend die Abziehschicht und die sich auf der Abziehschicht befindliche Klebeschicht und

iii) das aufeinander Laminieren der Komponenten aus i) und ii), so dass Deck- und Abziehschicht im Querschnitt des fertigen TTS die gegenüberliegenden, äußersten Schichten darstellen.

(15) Verfahren nach Gegenstand (14), umfassend

i) das Auftragen und gegebenenfalls anschließende Trocknen eines Films einer das Reservoir bildenden Zusammensetzung, gegebenenfalls in Form einer Lösung oder Dispersion in einem geeigneten Medium, auf die Seite der Deckschicht, welche als die der Haut zugewandten Seite bestimmt ist,

ii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf

die Abziehschicht und

iii) das aufeinander Laminieren der Komponenten aus i) und ii), so dass Deck- und Abziehschicht im Querschnitt des fertigen TTS die gegenüberliegenden, äußersten Schichten darstellen.

(16) Verfahren nach Gegenstand (14), umfassend

i) das Aufbringen und gegebenenfalls anschließende Trocknen eines Films einer das Reservoir bildenden Zusammensetzung, gegebenenfalls in Form einer Lösung oder Dispersion in einem geeigneten Medium, auf eine silikonisierte Abziehfolie (Liner) und das Kaschieren mit einer Deckschicht;

ii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf die Abziehschicht und

iii) das aufeinander Laminieren der Komponenten aus i) und ii), nach Abziehen von Liner, so dass Deck- und Abziehschicht im Querschnitt des fertigen TTS die gegenüberliegenden, äußersten Schichten darstellen.

(17) Verwendung eines Polymers oder Copolymers, das weder Hydroxylgruppen noch Carboxylgruppen aufweist, wobei diese Polymere oder Copolymere die Polymermatrix des Reservoirs darstellen, in die der Wirkstoff Rivastigmin eingebettet ist, zur Stabilisierung des Wirkstoffs Rivastigmin in einem transdermalen therapeutischen System, und/oder zur Verringerung des Abbaus des Wirkstoffs Rivastigmin in einem transdermalen therapeutischen System.

(18) Verwendung eines Polymers oder Copolymers nach Gegenstand (17), dadurch gekennzeichnet, dass Polymer oder Copolymer das weder Hydroxylgruppen noch Carboxylgruppen aufweist, ausgewählt ist aus der Gruppe bestehend aus Polyacrylaten, Acrylat-Vinylacetat Copolymeren, Polyisobutylen und Styrol-Butadien Copolymeren.

(19) Transdermales therapeutisches System nach einem der Gegenstände (1) bis (13) zur Verwendung in einem Verfahren zur Behandlung von Alzheimer und Parkinsondemenz.

Die Gegenstände (1) bis (19) können mit anderen in dieser Anmeldung beschriebenen Ausführungsformen kombiniert werden.

## Patentansprüche

**1.** Transdermales therapeutisches System zur Verabreichung eines Wirkstoffs durch die Haut umfassend:

a) eine Deckschicht,
b) ein auf der Deckschicht befindliches Reservoir umfassend eine den Wirkstoff enthaltende Polymermatrix,
c) eine auf dem Reservoir befindliche Klebeschicht umfassend einen Haftkleber und
d) eine auf der Klebeschicht befindliche Abziehschicht,

wobei der Wirkstoff Rivastigmin oder ein physiologisch verträgliches Salz, Hydrat oder Solvat davon ist, **dadurch gekennzeichnet, dass** die Polymermatrix des Reservoirs weder Hydroxylgruppen noch Carboxylgruppen aufweist, und das Reservoir kein Tocopherol, kein Butylhydroxdyanisol und kein Butylhydroxytoluol enthält.

**2.** Transdermales therapeutisches System nach Anspruch 1, **dadurch gekennzeichnet, dass** es keine Antioxidationsmittel, ausgewählt aus der Gruppe bestehend aus Tocopherolen und dessen Estern, Sesamol, dem Koniferylbenzoat des Benzoeharzes, Nordihydroguajakharz- und -guajaretsäure, Gallaten, Butylhydroxdyanisol, Ascorbinsäure und Salzen davon, Ascorbylpalmitat, Erythorbinsäure und Salzen davon, Monothioglycerol, Natriumformaldehydsulfoxylat, Natriummetabisulfit, Natriumbisulfit, Natriumsulfit, Kaliummetabisulfit, butyliertem Hydroxyanisol, butyliertem Hydroxytoluol und Propionsäure, enthält.

**3.** Transdermales therapeutisches System nach Anspruch 1 oder 2, wobei das Reservoir 20-30 Gew.-% Wirkstoff und 70-80 Gew.-% Polymermatrix, bezogen auf das Gesamtgewicht des Reservoirs, enthält.

**4.** Transdermales therapeutisches System nach einem der Ansprüche 1 bis 3, wobei die Klebeschicht zusätzlich einen

Gelbildner, ausgewählt aus der Gruppe bestehend aus hochdispersem Siliziumdioxid und pyrogener Kieselsäure, und einen Weichmacher, ausgewählt aus der Gruppe bestehend aus Mineralöl, Neutralöl, Paraffin, Polybuten, Leinsamenöl, Octylpalmitat, Squalen, Squalan, Silikonöl, Isobutylmyristat, Isostearylalkohol und Oleylalkohol, enthält.

**5.** Transdermales therapeutisches System nach Anspruch 4, wobei die Klebeschicht 60,0-74,9 Gew.-% Haftkleber, 0,1-2,0 Gew.-% Gelbildner und 25-39,9 Gew.-% Weichmacher, bezogen auf das Gesamtgewicht der Klebeschicht, enthält.

**6.** Transdermales therapeutisches System nach einem der Ansprüche 1 bis 5, wobei die Polymermatrix wenigstens ein Polymer und/oder Copolymer umfasst, ausgewählt aus der Gruppe bestehend aus Polyacrylaten, Acrylat-Vinylacetat Copolymeren, Polyisobutylen, Styrol-Butadien Copolymeren und Mischungen davon.

**7.** Transdermales therapeutisches System nach einem der Ansprüche 1 bis 6, **dadurch gekennzeichnet, dass** der Haftkleber Polyisobutylen ist.

**8.** Transdermales therapeutisches System nach einem der Ansprüche 1 bis 7, **dadurch gekennzeichnet, dass** das Polyisobutylen ein Gemisch aus zwei Polyisobutylenpolymeren mit unterschiedlichen Molekulargewichten ist.

**9.** Transdermales therapeutisches System nach Anspruch 8, **dadurch gekennzeichnet, dass** das erste Polyisobutylenpolymer ein mittleres Molekulargewicht $M_V$ von 40.000 g/mol hat, und das zweite Polyisobutylenpolymer ein mittleres Molekulargewicht $M_V$ von 400.000 g/mol hat.

**10.** Transdermales therapeutisches System nach Anspruch 8 oder 9, **dadurch gekennzeichnet, dass** das Gewichtsverhältnis des ersten Polyisobutylenpolymers zum zweiten Polyisobutylenpolymer 4:6 ist.

**11.** Transdermales therapeutisches System nach einem der Ansprüche 4 bis 10, **dadurch gekennzeichnet, dass** der Weichmacher Paraffin, Neutralöl, Mineralöl oder eine Mischung davon ist.

**12.** Transdermales therapeutisches System nach Anspruch 1, umfassend:

a) eine Deckschicht,
b) ein auf der Deckschicht befindliches Reservoir enthaltend 20 - 30 Gew.-% Wirkstoff und 70 - 80 % Polymermatrix, bezogen auf das Gesamtgewicht des Reservoirs, wobei die Polymermatrix im wesentlichen aus einem Acrylat-Vinylacetat-Copolymer ohne Hydroxylgruppen und ohne Carboxylgruppen besteht;
c) eine auf dem Reservoir befindliche Klebeschicht umfassend 0,1 - 1 Gew.-% Siliziumdioxid, 25 - 39.9 Gew.-% Paraffinum perliquidum (Ph. Eur.) und 60 - 79,9 Gew.-% eines Gemisches aus Polyisobutylen mit einem mittleren Molekulargewicht $M_V$ von 40.000 g/mol und Polyisobutylen mit einem mittleren Molekulargewicht $M_V$ von 400.000 g/mol; und
d) eine auf der Klebeschicht befindliche Abziehschicht.

**13.** Verfahren zur Herstellung eines transdermalen therapeutischen Systems nach einem der Ansprüche 1 bis 12, umfassend

i) die Herstellung einer das Reservoir enthaltenden Komponente, enthaltend die Deckschicht und das Reservoir, welches sich auf der Seite der Deckschicht befindet, welche als die der Haut zugewandten Seite bestimmt ist,
ii) die Herstellung einer die Klebeschicht enthaltenden Komponente, enthaltend die Abziehschicht und die sich auf der Abziehschicht befindliche Klebeschicht und
iii) das aufeinander Laminieren der Komponenten aus i) und ii), so dass Deck-und Abziehschicht im Querschnitt des fertigen TTS die gegenüberliegenden, äußersten Schichten darstellen.

**14.** Verfahren nach Anspruch 13, umfassend

i) das Auftragen und gegebenenfalls anschließende Trocknen eines Films einer das Reservoir bildenden Zusammensetzung, gegebenenfalls in Form einer Lösung oder Dispersion in einem geeigneten Medium, auf die Seite der Deckschicht, welche als die der Haut zugewandten Seite bestimmt ist,
ii) das Auftragen und gegebenenfalls anschließende Trocknen eines Films einer die Klebeschicht bildenden Zusammensetzung, gegebenenfalls in Form einer Lösung oder Dispersion in einem geeigneten Medium, auf

die Abziehschicht und
iii) das aufeinander Laminieren der Komponenten aus i) und ii), so dass Deck-und Abziehschicht im Querschnitt des fertigen TTS die gegenüberliegenden, äußersten Schichten darstellen.

**15.** Verwendung eines Polymers oder Copolymers, das weder Hydroxylgruppen noch Carboxylgruppen aufweist, wobei diese Polymere oder Copolymere die Polymermatrix des Reservoirs darstellen, in die der Wirkstoff Rivastigmin eingebettet ist, zur Stabilisierung des Wirkstoffs Rivastigmin und/oder zur Verringerung des Abbaus des Wirkstoffs Rivastigmin in einem transdermalen therapeutischen System nach Anspruch 1.

**16.** Verwendung nach Anspruch 15, **dadurch gekennzeichnet, dass** das Polymer oder Copolymer, das keine Hydroxylgruppen und keine Carboxylgruppen enthält, ausgewählt ist aus der Gruppe bestehend aus Polyacrylaten, Acrylat-Vinylacetat Copolymeren, Polyisobutylen und Styrol-Butadien Copolymeren.

**17.** Transdermales therapeutisches System nach einem der Ansprüche 1 bis 12 zur Verwendung in einem Verfahren zur Behandlung von Alzheimer und Parkinsondemenz.

## Claims

**1.** A transdermal therapeutic system for administering an active substance through the skin comprising:

a) a cover layer,
b) a reservoir present on the cover layer, comprising a polymer matrix containing the active substance,
c) an adhesive layer present on the reservoir comprising a contact adhesive, and
d) a removable layer present on the adhesive layer,

wherein the active substance is rivastigmine or a physiologically tolerable salt, hydrate or solvate thereof, **characterized in that** the polymer matrix of the reservoir includes neither hydroxyl groups nor carboxyl groups, and the reservoir does not contain tocopherol, butylhydroxdyanisol and butylhydroxytoluol.

**2.** The transdermal therapeutic system of claim 1, **characterized in that** it does not contain any antioxidants selected from the group consisting of tocopherols and their esters, sesamol, the coniferyl benzoate of benzoin, nordihydroguaiaretic resin and -guaiaretic acid, gallates, butylhydroxdyanisole, ascorbic acid and salts thereof, ascorbylpalmitate, erythorbic acid and salts thereof, monothioglycerol, sodium formaldehyde sulfoxylate, sodium metabisulphite, sodium bisulphite, sodium sulphite, potassium metabisulphite, butylated hydroxyanisole, butylated hydroxytoluene and propionic acid.

**3.** The transdermal therapeutic system of claim 1 or 2, wherein the reservoir contains 20-30 wt% active substance and 70-80 wt% polymer matrix in relation to the total weight of the reservoir.

**4.** The transdermal therapeutic system of any one of claims 1 to 3, wherein the adhesive layer additionally contains a gelling agent selected from the group consisting of highly dispersed silicon dioxide and pyrogenic silica, and an emollient selected from the group consisting of mineral oil, neutral oil, paraffin, polybutene, linseed oid, octyl palmitate, squalene, squalane, silicone oil, isobutyl myristate, isostearyl alcohol and oleyl alcohol.

**5.** The transdermal therapeutic system of claim 4, wherein the adhesive layer contains 60.0 to 74.9 wt% contact adhesive, 0.1 to 2.0 wt% gelling agent and 25 to 39.9 wt% emollient in relation to the total weight of the adhesive layer.

**6.** The transdermal therapeutic system of any one of claims 1 to 5, wherein the polymer matrix comprises at least one polymer and/or copolymer selected from the group consisting of polyacrylates, acrylate-vinyl acetate copolymers, polyisobutylene, styrene-butadiene copolymers and mixtures thereof.

**7.** The transdermal therapeutic system of any one of claims 1 to 6, **characterized in that** the contact adhesive is polyisobutylene.

**8.** The transdermal therapeutic system of any one of claims 1 to 7, **characterized in that** the polyisobutylene is a mixture of two polyisobutylene polymers of different molecular weights.

**9.** The transdermal therapeutic system of claim 8, **characterized in that** the first polyisobutylene polymer has a mean molecular weight Mv of 40,000 g/mol, and the second polyisobutylene polymer has a mean molecular weight $M_V$ of 400,000 g/mol.

**10.** The transdermal therapeutic system of claim 8 or 9, **characterized in that** the weight ratio of the first polyisobutylene polymer in relation to the second polyisobutylene polymer is 4:6.

**11.** The transdermal therapeutic system of any one of claims 4 to 10, **characterized in that** the emollient is paraffin, neutral oil, mineral oil or a mixture thereof.

**12.** The transdermal therapeutic system of claim 1 comprising

a) a cover layer,
b) a reservoir present on the cover layer containing 20-30 wt% active substance and 70-80 wt% polymer matrix in relation to the total weight of the reservoir, wherein the polymer matrix essentially consists of an acrylate-vinyl acetate copolymer without hydroxyl groups and without carboxyl groups;
c) an adhesive layer present on the reservoir comprising 0.1-1 wt% silicon dioxide, 25-39.9 wt% paraffinum perliquidum (Ph. Eur.) and 60-79.9 wt% of a mixture of polyisobutylene having a mean molecular weight $M_V$ of 40,000 g/mol, and polyisobutylene having a mean molecular weight Mv of 400,000 g/mol; and
d) a removable layer present on the adhesive layer.

**13.** A method for preparing the transdermal therapeutic system of anyone of claims 1 to 12 comprising

i) preparing a component containing the reservoir, comprising the cover layer and the reservoir, which is located on the side of the cover layer that is intended as the side to be directed toward the skin,
ii) preparing a compound containing the adhesive layer made up of the removable layer and the adhesive layer located on the removable layer and
iii) laminating to combine the components from i) and ii) so that in the end, in the cross-section of the completed TTS, the cover and removable layers constitute the outermost, opposite layers.

**14.** The method of claim 13 comprising

i) applying, and optionally subsequently drying, a film of a composition forming the reservoir, optionally in form of a solution or dispersion in a suitable medium, onto the side of the cover layer that is to be directed toward the skin,
ii) applying, and optionally subsequently drying, a film of a composition forming the adhesive layer, optionally in the form of a solution or dispersion in a suitable medium, to the removable layer, and
iii) laminating together the components from i) and ii) in order for the cover layer and the removable layer to constitute in the cross-section of the completed TTS the outermost, opposite layers.

**15.** Use of a polymer or copolymer having neither hydroxyl groups nor carboxyl groups, wherein these polymers or copolymers constitute the polymer matrix of the reservoir, in which the active substance rivastigmine is embedded, for stabilizing the active substance rivastigmine and/or reducing the degradation of the active substance rivastigmine in the transdermal therapeutic system of claim 1.

**16.** The use of claim 15, **characterized in that** the polymer or copolymer containing no hydroxyl groups and no carboxyl groups is selected from the group consisting of polyacrylates, acrylate-vinyl acetate copolymers, polyisobutylene and styrene-butadiene copolymers.

**17.** The transdermal therapeutic system of anyone of claims 1 to 12 for use in a method for the treatment of Alzheimer's disease and Parkinson's dementia.

**Revendications**

**1.** Système thérapeutique transdermique servant à administrer un principe actif par voie cutanée, comprenant :

a) une couche de recouvrement,

b) un réservoir se trouvant sur la couche de recouvrement comprenant une matrice polymère contenant le principe actif,
c) une couche de colle se trouvant sur le réservoir, comprenant un adhésif et
d) une couche pelable se trouvant sur la couche de colle,

dans lequel le principe actif est de la rivastigmine ou un sel, un hydrate ou un solvate de cette dernière physiologiquement compatible,
**caractérisé en ce que** la matrice polymère du réservoir ne présente aucun groupe hydroxyle ni aucun groupe carboxyle, et le réservoir ne contient aucun tocophérol, aucun butylhydroxyanisole, ni aucun hydroxytoluène butylé.

**2.** Système thérapeutique transdermique selon la revendication 1, **caractérisé en ce qu'**il ne contient aucun antioxydant choisi parmi le groupe constitué des tocophérols et de leurs esters, du sésamol, du benzoate de coniféryle de la résine de benzoïne, de la résine acide de nordihydro-guaïac et de l'acide nordihydroguaiarétique, de gallates, du butylhydroxyanisole, de l'acide ascorbique et de ses sels, du palmitate d'ascorbyle, de l'acide érythorbique et de ses sels, du monothioglycérol, du sulphoxylate formaldéhyde de sodium, du métabisulfite de sodium, du bisulfite de sodium, du sulfite de sodium, du métabisulfite de potassium, de l'hydroxyanisole butylé, de l'hydroxytoluène butylé et de l'acide propionique.

**3.** Système thérapeutique transdermique selon la revendication 1 ou 2, dans lequel le réservoir contient 20 - 30 % en poids de principe actif et 70 - 80 % en poids de matrice polymère par rapport au poids total du réservoir.

**4.** Système thérapeutique transdermique selon l'une quelconque des revendications 1 à 3, dans lequel la couche de colle contient en supplément un agent gélifiant, choisi parmi le groupe constitué d'un dioxyde de silicium à dispersion élevée et de l'acide silicique pyrogène, et un plastifiant choisi parmi le groupe constitué d'huile minérale, d'huile neutre, de paraffine, de polybutène, d'huile de lin, d'octyl palmitate, de squalène, de squalane, d'huile de silicone, d'isobutylmyristate, d'alcool isostéarylique et d'alcool oléylique.

**5.** Système thérapeutique transdermique selon la revendication 4, dans lequel la couche de colle contient 60,0 - 74,9 % en poids d'adhésif, 0,1 - 2,0 % en poids d'agent gélifiant et 25 - 39,9 % en poids de plastifiant, par rapport au poids total de la couche de colle.

**6.** Système thérapeutique transdermique selon l'une quelconque des revendications 1 à 5, dans lequel la matrice polymère comprend au moins un polymère et/ou un copolymère choisi parmi le groupe constitué de polyacrylates, de copolymères d'acrylate-acétate de vinyle, de polyisobutylène, de copolymères de styrène-butadiène et de mélanges de ces derniers.

**7.** Système thérapeutique transdermique selon l'une quelconque des revendications 1 à 6, **caractérisé en ce que** l'adhésif est du polyisobutylène.

**8.** Système thérapeutique transdermique selon l'une quelconque des revendications 1 à 7, **caractérisé en ce que** le polyisobutylène est un mélange composé de deux polymères de polyisobutylène présentant des poids moléculaires différents.

**9.** Système thérapeutique transdermique selon la revendication 8, **caractérisé en ce que** le premier polymère de polyisobutylène est doté d'un poids moléculaire moyen Mv de 40.000 g/mol, et le deuxième polymère de polyisobutylène est doté d'un poids moléculaire moyen Mv de 400.000 g/mol.

**10.** Système thérapeutique transdermique selon la revendication 8 ou 9, **caractérisé en ce que** le rapport de poids entre le premier polymère de polyisobutylène et le deuxième polymère de polyisobutylène est de 4:6.

**11.** Système thérapeutique transdermique selon l'une quelconque des revendications 4 à 10, **caractérisé en ce que** le plastifiant est de la paraffine, de l'huile neutre, de l'huile minérale ou un mélange de ces dernières.

**12.** Système thérapeutique transdermique selon la revendication 1, comprenant :

a) une couche de recouvrement,
b) un réservoir se trouvant sur la couche de recouvrement contenant 20 - 30 % en poids de principe actif et 70 - 80 % en poids de matrice polymère par rapport au poids total du réservoir, dans lequel la matrice polymère

est constituée sensiblement d'un copolymère d'acrylate-acétate de vinyle sans groupes hydroxyle et sans groupes carboxyle ;

c) une couche de colle se trouvant sur le réservoir, comprenant 0,1 - 1 % en poids de dioxyde de silicium, 25 - 39,9 % en poids de paraffinum perliquidum (Ph. Eur.) et 60 - 79,9 % en poids d'un mélange composé de polyisobutylène présentant un poids moléculaire moyen Mv de 40.000 g/mol et de polyisobutylène présentant un poids moléculaire moyen Mv de 400.000 g/mol ; et

d) une couche pelable se trouvant sur la couche de colle.

**13.** Procédé servant à fabriquer un système thérapeutique transdermique selon l'une quelconque des revendications 1 à 12, comprenant

i) la fabrication d'une composante contenant le réservoir, contenant la couche de recouvrement et le réservoir, qui se trouve sur le côté de la couche de recouvrement, qui est définie en tant que le côté tourné vers la peau,

ii) la fabrication d'une composante contenant la couche de colle, contenant la couche pelable et la couche de colle se trouvant sur la couche pelable ; et

iii) le laminage les unes sur les autres des composantes issues de i) et de ii) de sorte que la couche de recouvrement et la couche pelable constituent dans la section transversale du STT fini les couches situées le plus à l'extérieur se faisant face.

**14.** Procédé selon la revendication 13, comprenant

i) l'application et éventuellement le séchage consécutif d'un film d'une composition formant le réservoir, éventuellement sous la forme d'une solution ou d'une dispersion dans un milieu adapté sur le côté de la couche de recouvrement, qui est définie en tant que le côté tourné vers la peau ;

ii) l'application et éventuellement le séchage consécutif d'un film d'une composition formant la couche de colle, éventuellement sous la forme d'une solution ou d'une dispersion dans un milieu adapté, sur la couche pelable ; et

iii) le laminage les unes après les autres des composantes issues de i) et de ii) de sorte que la couche de recouvrement et la couche pelable constituent, dans la section transversale du STT fini, les couches situées le plus à l'extérieur, se faisant face.

**15.** Utilisation d'un polymère ou d'un copolymère, qui ne présente ni un groupe hydroxyle ni un groupe carboxyle, dans laquelle lesdits polymères et copolymères constituent la matrice polymère du réservoir, dans laquelle est incorporé le principe actif de la rivastigmine, aux fins de la stabilisation du principe actif de la rivastigmine et/ou aux fins de la réduction de l'élimination du principe actif de la rivastigmine dans un système thérapeutique transdermique selon la revendication 1.

**16.** Utilisation selon la revendication 15, **caractérisée en ce que** le polymère ou le copolymère, qui ne contient aucun groupe hydroxyle ni aucun groupe carboxyle, est choisi parmi le groupe constitué de polyacrylates, de copolymères d'acrylate-acétate de vinyle, de polyisobutylènes et de copolymères de styrène-butadiène.

**17.** Système thérapeutique transdermique selon l'une quelconque des revendications 1 à 12 destiné à être utilisé dans un procédé servant au traitement de la maladie d'Alzheimer et de la maladie de Parkinson.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 2203040 A
- WO 0203969 A
- DE 19918106
- WO 2007064407 A1
- US 20080044461 A1
- US 20070259028 A1
- US 20040086552 A1
- US 6689379 B1
- EP 1047409 A
- WO 03017988 A1
- WO 03017988 A